# NEUE EUROPÄISCHE PATENTSCHRIFT

(11) **EP 0 318 679 B2**
(45) Veröffentlichungstag und Bekanntmachung des Hinweises auf die Entscheidung über den Einspruch: **19.07.1995**
(45) Hinweis auf die Patenterteilung: 05.02.1992
(21) Anmeldenummer: 88116801.7
(22) Anmeldetag: 10.10.1988
(51) Int. Cl.: A61F 2/34

(54) **Endoprothese für eine Hüftgelenkspfanne**
Endoprosthesis for an acetabular cup
Endoprothèse pour un cotyle de hanche

(30) Priorität: 09.11.1987 CH 4364/87
(43) Veröffentlichungstag der Anmeldung: 07.06.1989
(73) Patentinhaber: Intraplant AG, CH-6330 Cham (CH)
(72) Erfinder: Imhof, Martin, CH-6343 Rotkreuz (CH)
(74) Vertreter: Patentanwälte Schaad, Balass, Menzl & Partner AG

(56) Entgegenhaltungen:
- EP-A- 0 065 482
- DE-A- 524 051
- DE-A- 2 834 296
- DE-A- 2 950 536
- DE-A- 3 446 048
- DE-U- 8 500 867
- K. Bläsius, E. Schneider, Endoprothesen- Atlas Hüfte, Stuttgart New York 1989, Seiten 31 und 93
- Dubbel:Taschenbuch für den Maschinenbau, Springer Verlag, 17. Auflage, S. G36

## Beschreibung

Die vorliegende Erfindung betrifft eine Endoprothese gemäss dem Oberbegriff des Patentanspruches 1. Eine solche Endoprothese ist aus der EP-A-0 065 482 bekannt.

Dabei sei bemerkt, dass auch im vorliegenden Zusammenhang unter "selbstschneidendem Gewinde" ein solches zu verstehen ist, bei dem die Gewindegänge pro Umlauf oder Windung durch mehrere Lücken in Abschnitte unterteilt ist, wobei jeweils die in Einschraubrichtung gesehen nachlaufende Wand jeder Lücke eine Schneidfläche bildet,deren Schneidenform durch das Gewindeprofil gegeben ist.

Endoprothesen der eingangs genannten Art sind bekannt. Die Form der Mantelhülse bringt es mit sich, dass auch das selbstschneidende Gewinde gewissermassen einem konischen Aussengewinde vergleichbar ist, Bei den bekannten Endoprothesen hat das selbstschneidende Gewinde das Profil etwa eines Spitzgewindes oder eines Trapezgewindes, wobei die Höhe des Gewindes in allen Abschnitten etwa konstant ist.

Da, wie gesagt, das Gewinde einem konischen Gewinde vergleichbar ist und bei den bekannten Endoprothesen die Gewindehöhe in allen Abschnitten konstant ist, hat dies zur Folge, dass beim Einschrauben der Mantelhülse in die Knochensubstanz die auf die Schneiden folgenden Abschnitte des Gewindes auf die Knochensubstanz sowohl in radialer als auch in axialer Richtung einen Druck ausüben, als ob in die Kehlen des in die Knochensubstanz geschnittenen "Muttergewindes" in Umfangsrichtung Keile eingetrieben würden. Dies hat zur Folge, dass zum Einschrauben ein vergleichsweise hohes Drehmoment aufzuwenden ist und daher der operierende Chirurg nicht immer genau spürt, wann die Mantelhülse ausreichend tief eingeschraubt ist. Ebenso kann bei den bekannten Endoprothesen das Risiko eines Risses in der Knochensubstanz infolge des genannten Druckes nicht ganz ausgeschlossen werden, wobei dieser Riss nicht notwendigerweise während der Implantation geschieht, sondern - was schlimmer ist - wenn nach dem Eingriff begonnen wird, das implantierte Hüftgelenk wieder der normalen Belastung auszusetzen. Eine unvollständig eingeschraubte Mantelhülse und/oder eine Rissbildung in der Knochensubstanz haben aber einen lockeren Sitz der implantierten Hüftgelenkspfanne zur Folge mit möglicherweise der Konsequenz eines erneuten chirurgischen Eingriffes.

Es ist daher eine Aufgabe der Erfindung, eine Endoprothese der eingangs genannten Art zu schaffen, die vergleichweise leicht in eine vorbereitete Vertiefung in der Knochensubstanz einzuschrauben ist und von der beim Einschrauben keine Druckwirkung auf die Knochensubstanz ausgeübt wird.

Diese Aufgabe wird von der vorgeschlagenen Endoprothese dadurch gelöst, dass sie die im Kennzeichen des Patentanspruches 1 angegebenen Merkmale aufweist. Durch den Umstand, dass das Gewinde ein Flachgewinde ist, üben die auf die Schneidflächen folgenden Abschnitte der Gewindeflanken keinen axialen Druck auf die Flanken des in die Knochensubstanz eingeschnittenen "Muttergewindes" aus. Dasselbe gilt für die auf die Schneiden folgenden Abschnitte des Gewindes, die dank der Hinterdrehung bzw. dank dem Hinterschnitt keinen radialen Druck auf den Grund des in die Knochensubstanz eingeschnittenen Muttergewindes ausüben.

Merkmale bevorzugter Ausführungsformen sind in den abhängigen Ansprüchen umschrieben.

Nachstehend ist anhand der Zeichnung ein Beispiel der erfindungsgemässen Endoprothese beschrieben.
Es zeigt :
- Fig. 1: rechts eine Seitenansicht, links einen Axialschnitt durch die Mantelhülse einer Endoprothese ; und
- Fig. 2: eine Ansicht aus Richtung des Pfeiles II der Fig. 1.

In der Zeichnung ist von einer Endoprothese lediglich die Mantelhülse 10 gezeigt, die im wesentlichen die Form eines zu seinem Polbereich hin sich verjüngenden Rotationskörpers hat, der eine nur schematisch dargestellte Höhlung 11 aufweist, die zur Aufnahme einer nicht dargestellten Lagerpfanne aus einem Kunststoff, beispielsweise aus einem Polyäthylen bestimmt ist. Nicht dargestellt sind ebenfalls die in der Höhlung 11 allenfalls vorhandenen Kerben zur Verankerung der Lagerpfanne und die beispielsweise in der Grundfläche 12 der Mantelhülse 10 vorhandenen Bohrungen oder dergleichen zum Ansetzen eines Einschraubwerkzeuges.

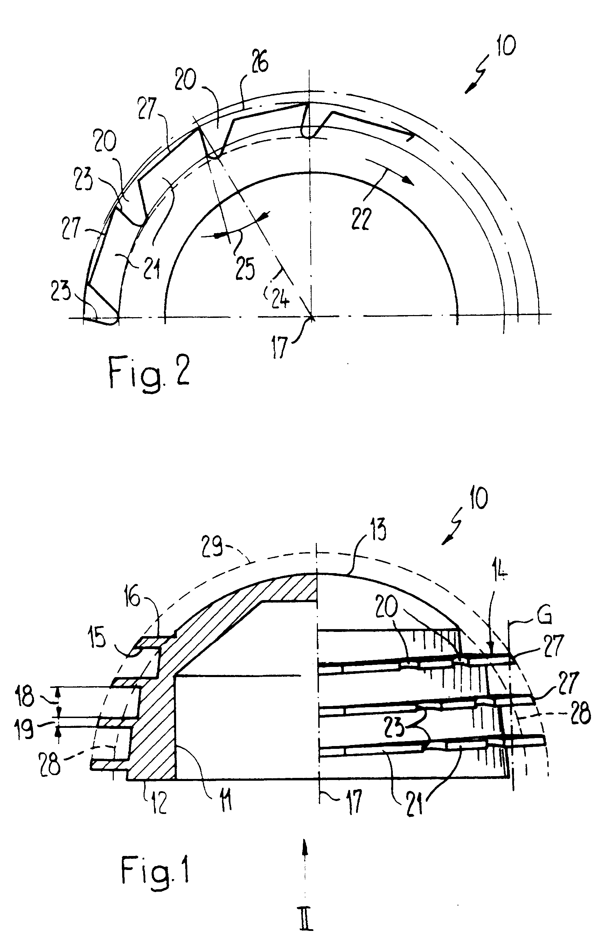

Die Aussenform der Mantelhülse ist in diesem Beispiel jener eines "Stufenkegels" vergleichbar, dessen Scheitel- oder Polbereich 13 durch eine Kugelkalotte gebildet ist. Der Stufenkegel trägt auf seiner Aussenseite ein selbstschneidendes Gewinde 14. Wie der Fig. 1 links zu entnehmen ist, hat das Gewinde das Profil eines Flachgewindes, wobei die Erzeugenden der Flanken 15 und 16 rechtwinklig zu der mit 17 bezeichneten Axe der Mantelhülse 10 ist, Der Abstand 18 von Gewindegang zu Gewindegang ist konstant und beträgt ein Mehrfaches der Dicke 19 jedes Gewindeganges. Ebenso ist die Dicke 19 jedes Gewindeganges konstant. Schliesslich ist der Stegungswinkel der sich verjüngenden Wendelflächen der Flanken 15, 16 an der Durchstoss-Stelle einer zur Axe 17 der Mantelhülse 10 parallelen Geraden (strichpunktiert und in Fig. 1 mit G bezeichnet) konstant.

Wie bei jedem selbstschneidenden Gewinde ist, wie der Fig. 1 rechts und der Fig. 2 links zu entnehme ist, jeder Gewindegang durch Lücken 20 in Abschnitte 21 aufgeteilt. Die in Einschraubrichtung (Pfeil 22) gesehen nachlaufende Seitenfläche jeder der Lücken 20 bildet somit eine Schneidfläche 23, die gegenüber einer durch die Axe 17 gelegte Radialebene 24 einen "Spanwinkel" 25, beispielsweise in der Grössenordnung zwischen 15° und 30° aufweist.

Da das Gewinde 14 radial von einem sich verjüngenden Grundkörper absteht, ist die Hüllinie des Scheitels der Gänge des Gewindes 14 nicht ein Kreis, sondern - in Richtung der Achse 17 projiziert - eine Spirale. Diese Spirale ist in Fig. 2 mit der strichpunktierten Linie 26 angedeutet. Wie der Fig. 2 zu entnehmen ist, sind die Scheitelflächen 27 aller auf eine der Schneidflächen 23 folgenden Abschnitte 21 des Gewindes 14 gegenüber dieser Spirale 26 hinterschnitten. Diese Scheitelflächen können auch hinterdreht sein.

Aus dem Gesagten ergibt sich, dass unabhängig davon, wie weit die Mantelhülse 10 schon eingeschraubt ist, es immer nur der Scheitelbereich der jeweiligen Schneidfläche 23 ist, die tatsächlich sich in die Knochensubstanz der entsprechend vorbereiteten Vertiefung eingräbt. Da es sich um ein Flachgewinde handelt üben die Flanken 15 und 16 keinen axialen Druck auf die Knochensubstanz aus und da ferner die Scheitelflächen 27 hinterschnitten bzw. hinterdreht sind, üben diese auch keinen radialen Druck aus. Die beim Schneiden des "Muttergewindes" herausgeschnittene Knochensubstanz findet in den Lücken 20 Platz und verfestigt sich wieder nach vergleichsweise kurzer Zeit.

Zur Implantation der beschriebenen Mantelhülse 10:
Mit einem einem Krauskopf vergleichbaren Werkzeug wird in die Knochensubstanz eine Vertiefung in der Form einer Kugel kalotte eingearbeitet. Das Profil dieser Kugelkalotte ist mit der gestrichelten Linie 28 in Fig. 1 angedeutet und hat denselben Radius wie die Kugelkalotte des Scheitelbereiches 13 der Mantelhülse 10. Sodann kann die Mantelhülse 10 eingeschraubt werden, bis ihr Scheitelbereich 13 satt an den Scheitelbereich der in die Knochensubstanz eingearbeiteten Vertiefung anliegt. Dabei wird auf die Knochensubstanz keinerlei Keil- oder Druckwirkung ausgeübt. Das Einsetzen der Lagerpfanne (sofern noch nicht eingesetzt) erfolgt dann in der herkömmlichen Weise.

Zur Herstellung der beschriebenen Mantelhülse:
Ausgegangen wird in diesem Beispiel von einem Rohling, beispielsweise aus einer Titanlegierung in der Form einer Halbkugel, wie in Fig. 1 mit der gestrichelten Linie 29 angedeutet. Sodann wird die Höhlung 11 ausgedreht. Nun kann die Kalotte 13 abgedreht werden und/oder kann das Gewinde 14 geschnitten werden. Für das Gewinde 14 wird ein Profilstahl der Schnittbreite 18 verwendet, der je Umdrehung um die Distanz 18 + 19 vorgeschoben wird, während seine Zustellung in Richtung der Axe 17 in mehreren Stufen und in mehreren Durchgängen erfolgt, bis die gewünschte Gewindetiefe erreicht ist. Schliesslich werden, beispielsweise mit einem Profilfräser, die Lücken 20 gefräst, die beispielsweise jeweils entlang eines "Meridianes" verlaufen. Mit einem weiteren Fräser werden endlich die Scheitelflächen 27 gegenüber dem äussersten Abschnitt der zugeordneten Schneidfläche 23 hinterschnitten.

Die beschriebene Mantelhülse 10 gewährleistet einen satten Sitz in der Knochensubstanz, da die Flanken 15, 16 über die gesamte Länge des betreffenden Gewindeabschnittes 21 satt, jedoch sozusagen drucklos anliegen.

Während im vorliegenden Beispiel der Grundkörper der Mantelhülse 10 die Form eines durch eine Kugelkalotte abgeschlossenen Stufenkegels aufweist lässt sich das beschriebene Flachgewinde auf Grundkörpern mit einer beliebigen Erzeugenden verwirklichen, beispielsweise bei einer Erzeugenden eines Kreisbogens (Halbkugel), zweier Geraden (Kegelstumpf). Auch kann der Scheitel- oder Polbereich 13 eine aufgerauhte Oberflächenstruktur aufweisen, die bei der Regenerierung der Knochensubstanz das Anwachsen begünstigt. Schliesslich kann im Scheitelbereich der Mantelhülse auch eine durchgehende Oeffnung vorhanden sein.

## Patentansprüche

1. Endoprothese für eine Hüftgelenkspfanne mit einer zur Aufnahme eines Pfannenlagers bestimmten Mantelhülse (10), die die Form eines Rotationskörpers hat, der sich zu seinem Polbereich (13) hin verjüngt und der an seiner Aussenfläche ein selbstschneidendes Gewinde (14) trägt, wobei der Abstand (18) von Gang zu Gang des Gewindes (14) konstant ist und die an die mit ihren Endkanten auf einer Spiralwendel (26) liegenden Schneiden (23) anschliessenden Abschnitte (21) der Gewindegänge gegenüber dieser Spiralwendel hinterdreht oder hinterschnitten sind, dadurch gekennzeichnet, dass das Gewinde (14) ein Flachgewinde mit zueinander parallelen, rechtwinklig zur Achse (17) des Rotationskörpers verlaufenden Flanken (15, 16) ist.

2. Endoprothese nach Patentanspruch 1, dadurch gekennzeichnet, dass die Flankenhöhe des Gewindes (14) mehr als das Doppelte der Dicke (19) eines Gewindeganges ist.

3. Endoprothese nach Patentanspruch 1 oder 2, dadurch gekennzeichnet, dass der Abstand (18) von Gang zu Gang des Gewindes grösser ist als die Dicke (19) eines Gewindeganges.

4. Endoprothese nach einem der Patentansprüche 1 bis 3, dadurch gekennzeichnet, dass der Steigungswinkel aller Flanken (15, 16) des Gewindes an der Durchstoss-Stelle einer virtuellen, zur Axe (17) des Rotationskörpers parallelen Geraden (G) gleich gross ist.

5. Endoprothese nach einem der Patentansprüche 1 bis 4, dadurch gekennzeichnet, dass die Schneidflächen (23) der Schneiden des Gewindes (14) einen Spanwinkel (25) zwischen 10° und 30° aufweisen.

6. Endoprothese nach einem der Patentansprüche 1 bis 5, dadurch gekennzeichnet, dass die Hüllfläche der äussersten Abschnitte (27) der Schneidflächen (23) eine zur Axe des Rotationskörpers koaxiale Rotationsfläche ist.

## Claims

1. An endoprosthesis for an acetabulum comprising, to receive an acetabular bearing, a sleeve (10) which takes the form of a body of rotation which is tapered towards its pole region (13) and which has on its outer surface a self-tapping screw thread (14), whereby the distance (18) from one turn of the thread (14) to another is constant and the portions (21) of the thread turns which are adjacent the cutting edges (23) which have their end edges disposed on a spiral (26) are relieved or undercut in relation to this spiral, characterised in that the screw thread (14) is a flat thread, the flanks (15, 16) of which are parallel with one another and are at right-angles to the axis (17) of the body of rotation.

2. An endoprosthesis according to Patent Claim 1, characterised in that the flank height of the screw head (14) is more than twice the thickness (19) of one turn of the thread.

3. An endoprosthesis according to Patent Claim 1 or 2, characterised in that the distance (18) from turn to turn of the thread is greater than the thickness (19) of one turn of the thread.

4. An endoprosthesis according to one of Patent Claims 1 to 3, characterised in that the pitch angle of all flanks (15, 16) of the thread at the point of intersection is equal in size to a virtual straight line (G) parallel with the axis (17) of the body of rotation.

5. An endoprosthesis according to one of Patent Claims 1 to 4, characterised in that the cutting surfaces (23) of the cutting edges of the screw thread (14) have a cutting angle (25) of between 10° and 30°.

6. An endoprosthesis according to one of Patent Claims 1 to 5, characterised in that the addendum envelope of the outermost portions (27) of the cutting surfaces (23) is a surface of rotation coaxial with the axis of the body of rotation.

## Revendications

1. Endoprothèse pour un cotyle de hanche, comprenant une douille d'enveloppe (10) destinée à recevoir un coussinet et présentant la forme d'un corps de révolution qui se rétrécit en direction de la région de son pôle (13) et porte sur sa face extérieure un filet autotaraudeur (14), la distance (18) d'un pas de vis à l'autre du filet (14) étant constante, et les sections (21) des pas de vis raccordées aux tranchants (23) dont les arêtes terminales se situent sur une spirale (26) étant dépouillées ou contre-dépouillées par rapport à cette spirale, caractérisée en ce que le filet (14) est un filet carré comportant des flancs (15, 16) parallèles entre eux et orientés orthogonalement par rapport à l'axe (17) du corps de révolution.

2. Endoprothèse selon la revendication 1, caractérisée en ce que la hauteur des flancs du filet (14) est supérieure au double de l'épaisseur (19) d'un pas de vis.

3. Endoprothèse selon l'une des revendications 1 ou 2, caractérisée en ce que la distance (18) d'un pas de vis à l'autre du filet est plus grande que l'épaisseur (19) d'un pas de vis.

4. Endoprothèse selon l'une des revendications 1 à 3, caractérisée en ce que l'angle d'hélice de tous les flancs (15, 16) du filet, au point de percée d'une droite (G) virtuelle parallèle à l'axe (17) du corps de révolution, est constant.

5. Endoprothèse selon l'une des revendications 1 à 4, caractérisée en ce que les surfaces de coupe (23) des tranchants du filet (14) présentent un angle de coupe (25) compris entre 10° et 30°.

6. Endoprothèse selon l'une des revendications 1 à 5, caractérisée en ce que l'enveloppante des sections (27) des surfaces de coupe (23) situées le plus à l'extérieur, est une surface de révolution coaxiale à l'axe du corps de révolution.
